(19) Europäisches Patentamt
European Patent Office
Office européen des brevets

(11) EP 0 680 744 B1

(12) # FASCICULE DE BREVET EUROPEEN

(45) Date de publication et mention de la délivrance du brevet:
**29.09.1999 Bulletin 1999/39**

(51) Int. Cl.[6]: **A61K 7/06**

(21) Numéro de dépôt: **95400788.6**

(22) Date de dépôt: **07.04.1995**

(54) **Utilisation de flavonoides pour la protection des cheveux**

Verwendung von Flavonoiden zum Schutz der Haare

Use of flavonoid for hair protection

(84) Etats contractants désignés:
**DE ES FR GB IT**

(30) Priorité: **05.05.1994 FR 9405539**

(43) Date de publication de la demande:
**08.11.1995 Bulletin 1995/45**

(73) Titulaire: **L'OREAL**
**75008 Paris (FR)**

(72) Inventeurs:
- **Dubief, Claude**
**F-78150 Le Chesnay (FR)**
- **Braida-Valerio, Damarys**
**F-75004 Paris (FR)**

(74) Mandataire:
**Tezier Herman, Béatrice**
**L'OREAL-DPI**
**6 rue Bertrand Sincholle**
**92585 Clichy Cédex (FR)**

(56) Documents cités:

| | |
|---|---|
| **EP-A- 0 545 147** | **WO-A-94/14414** |
| **FR-A- 2 527 927** | **FR-A- 2 578 422** |
| **FR-A- 2 687 572** | **US-A- 4 603 046** |

- **S.T.N., Serveur de bases de données, KARLSRUHE, DE, Fichier Chemical Abstracts, vol 121, n 163714 * résumé * & JP-A-06179611 (DOWA YAKUSHO )**
- **DATABASE WPI Week 9309, Derwent Publications Ltd., London, GB; AN 93-071001 & JP-A-5 017 321 (KAWAGUCHI)**
- **DATABASE WPI Week 9434, Derwent Publications Ltd., London, GB; AN 94-277000 & KR-B-9 308 763 (PACIFIC CHEM IND CO)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Xerox (UK) Business Services
2.16.7/3.6

## Description

La présente invention a pour objet l'utilisation, dans une composition cosmétique, de flavonoïdes en tant qu'agents protecteurs des propriétés mécaniques de la kératine des cheveux.

Il est bien connu que les cheveux sont sensibilisés ou fragilisés à des degrés divers par l'action des agents atmosphériques et notamment de la lumière, ainsi que par l'action répétée des différents traitements capillaires tels que les permanentes, le défrisage, la teinture, la décoloration. De nombreuses publications divulguent que la lumière naturelle détruit certains aminoacides des cheveux. Ces agressions altérant la fibre capillaire, elles en diminuent les propriétés mécaniques comme la résistance à la traction, la charge à la rupture et l'élasticité, ou leur résistance au gonflement dans un milieu aqueux.

La résistance à la traction peut être mesurée par le palier à 15% d'extension. Le palier à 15% d'extension est la force qu'il faut appliquer à un cheveu mouillé d'une longueur donnée pour l'allonger de façon permanente de 15%. Plus cette force est élevée, plus le cheveu est élastique et résistant.

Pour lutter contre la dégradation mécanique de la kératine des cheveux par la lumière, on a déjà proposé d'utiliser certaines substances susceptibles de filtrer les radiations lumineuses, comme l'acide 2-hydroxy-4-méthoxy benzophénone-5-sulfonique ou ses sels (FR-A-2 627 085) ou l'acide 4-(2-oxo-3-bornylidène méthyl) benzène sulfonique ou ses sels (EP-A-329 032) ou encore la lactoferrine (FR-A-2 673 839).

Or, la demanderesse a maintenant découvert, de façon inattendue et surprenante, que les flavonoïdes peuvent également préserver et/ou renforcer les propriétés mécaniques des cheveux, et notamment leur résistance à la traction et leur élasticité, face aux différentes agressions susceptibles d'être subies par ces derniers.

On notera que certains flavonoïdes sont connus pour leur utilisation dans la préparation de compositions cosmétiques, à titre d'agents de protection de la peau et/ou de ses phanères contre l'oxygène singulet, tel que cela est décrit dans la demande de brevet FR-A-2 687 752. Toutefois, ce document reste muet sur les effets techniques particuliers qui pourraient être obtenus par application desdits flavonoïdes sur les cheveux, en particulier lorsque ceux-ci sont soumis à l'action répétée de traitements capillaires.

Le document US 4 603 046 décrit des compositions pour le traitement de la peau, telles que des compositions solaires ayant des compositions ayant des propriétés filtrantes pour les UV ou des compositions ayant des propriétés hydratantes. Ces compositions contiennent de la trihydroxyalkylrutoside, de préférence de la troxérutine.

L'abrégé du document KR 9 308 763 décrit une composition cosmétique contenant du gallate d'épigallocatéchine, de la galanguine et de l'acide phytique. La composition est utilisée pour la peau et les cheveux pour supprimer des radicaux libres et les espèces activées de l'oxygène.

La demande WO94/14414 concerne une composition cosmétique qui contient un système antioxydant constitué par l'association d'un extrait de ginkgo et d'au moins un composé phénolique et l'utilisation de cette composition pour prévenir et traiter les dommages cellulaires sur la peau ou le cuir chevelu provoqué par des radicaux libres.

Dans ces documents, il n'est ni décrit ni suggéré d'utiliser des flavonoïdes pour préserver ou renforcer les propriétés mécaniques des cheveux et notamment leur résistance à la traction et leur élasticité.

Le document FR 2 527 927 vise l'amélioration des propriétés cosmétiques de la chevelure au moyen d'une composition contenant un dérivé de flavonol. Il s'agit d'améliorer la douceur au toucher, l'aptitude au démêlage, le caractère lisse ou gonflant de la coiffure.

La présente invention a donc pour objet l'utilisation, dans une composition cosmétique, de flavonoïdes pour préserver et/ou de renforcer au moins une propriété mécanique des cheveux choisie parmi la résistance à la traction, l'élasticité, la résistance au gonflement dans un milieu aqueux ou la charge à la rupture, face à l'action répétée de traitements capillaires, les flavonoïdes étant choisis parmi les flavanones, les flavones, les flavonols, les dihydroflavonols, les catéchines et les leucoanthocyanidines.

Selon l'invention, on utilise à titre préférentiel les flavonoïdes répondant à la formule générale (I) :

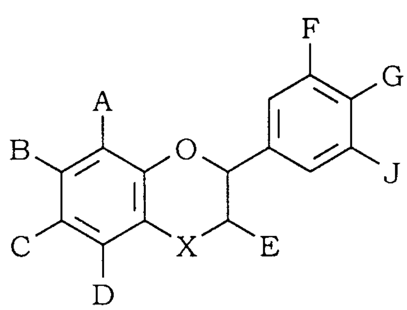

(I)

ou (II):

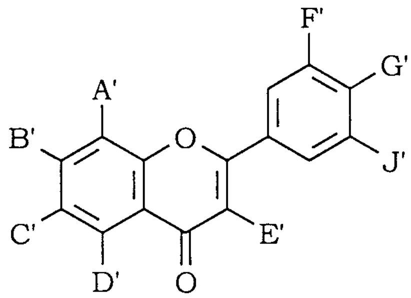

(II)

dans lesquelles :

A, B, C et D, indépendamment l'un de l'autre, représentent H ou OH, E représente H, OH, ou OR, où R représente :

R = 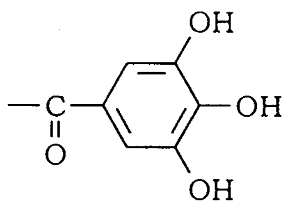

F, G, J représentent, indépendamment l'un de l'autre, H ou OH; et X représente :

X = 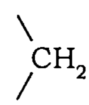 , 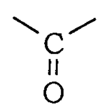 , 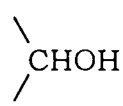

A', C' et D', indépendamment l'un de l'autre, représentent H, OH ou $OCH_3$,
E' repésente H , OH ou OR', où R' représente un radical d'un sucre,
B', F', G' et J', indépendamment l'un de l'autre, représentent H, OH, $OCH_3$ ou $OCH_2$-$CH_2$-OH.

De préférence, R' représente le radical 6-O-(6-déoxy-α-L-mannopyranosyl)-β-D-glucopyranosyl.

Les composés de formule (I) et (II) peuvent être obtenus notamment selon les procédés décrits dans "The Flavonoïds" Harborne J.B., Mabry T.J., Helga Mabry, 1975, pages 1 à 45.

Dans le cadre de la présente invention, les flavonoïdes plus particulièrement préférés sont choisis parmi la taxifoline, la catéchine, l'épicatéchine, l'ériodictyol, la naringénine, la rutine, la troxérutine, la chrysine, la tangérétine, la lutéoline, l'épigallocatéchine et le gallate de l'épigallocatéchine.

Dans les compositions pour l'utilisation selon l'invention, les composés de formule (I) et (II) sont généralement présents à une concentration comprise entre 0, 001 et 10 % en poids et de préférence entre 0, 005 et 5 % en poids par rapport au poids total de la composition.

Les compositions peuvent se présenter sous forme de lotion aqueuse ou hydroalcoolique, monophasique ou polyphasique, de gel monophasique ou polyphasique, d'émulsion, de crème, de dispersion vésiculaire, de mousse, de spray.

Les compositions capillaires peuvent se présenter sous forme de shampooing, d'après-shampooing à rincer ou non, de mousse de coiffage, de compositions pour permanente, défrisage, coloration ou décoloration, ou encore sous forme de compositions à rincer, à appliquer avant ou après une coloration, une permanente ou un défrisage ou encore entre les deux étapes d'une permanente ou d'un défrisage.

Les compositions peuvent par ailleurs contenir des additifs cosmétiques conventionnels choisis parmi les

corps gras, les solvants organiques, les silicones, les épaississants, les adoucissants, les tensio-actifs, les polymères anioniques, cationiques, non-ioniques ou amphotères, les agents anti-mousses, les agents conditionneurs du cheveu tels que des protéines, des vitamines, les agents traitants (agents anti-chute, antipelliculaires), les céramides tels que ceux cités dans EP-A-500 437, les colorants, les agents nacrants, les filtres solaires et notamment les filtres sulfoniques, les parfums, les conservateurs, les agents antimicrobiens, les électrolytes, les agents stabilisants tels que l'acide érythorbique et le métabisulfite de sodium, les agents séquestrants, les agents propulseurs.

Plus précisément, comme corps gras, on peut utiliser une huile ou une cire ou leur mélange, des acides gras, des alcools gras, des esters d'acides gras tels que les triglycérides d'acide gras en $C_6$ à $C_{18}$, de la vaseline, de la paraffine, de la lanoline, de la lanoline hydrogénée ou acétylée.

Parmi les huiles, on peut citer les huiles minérales, animales, végétales ou les huiles de synthèse, et notamment l'huile de vaseline, de paraffine, de ricin, de jojoba, de sésame, ainsi que les huiles et les gommes de silicone, les isoparaffines et les huiles fluorées ou perfluorées.

Parmi les cires, on peut citer les cires animales, végétales, minérales ou de synthèse, et notamment les cires d'abeilles, de Candelila, les ozokérites, les cires microcristallines ainsi que les cires et résines de silicone.

Parmi les solvants organiques usuellement utilisés dans les compositions cosmétiques, on peut citer plus précisément les mono-alcools ou polyalcools inférieurs en $C_1$ à $C_6$ comme l'éthanol, l'isopropanol, l'éthylèneglycol, le diéthylènegycol, le propylèneglycol, le glycérol.

Les agents épaississants peuvent être choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, la gomme de xanthane, les scléroglucanes, les acides polyacryliques réticulés.

Comme agents tensio-actifs et comme polymères , on peut utiliser tous ceux bien connus de l'état de la technique notamment pour leur utilisation dans des compositions capillaires.

De préférence, les compositions pour l'utilisation selon l'invention, ne contiennent pas d'extrait de ginkgo.

Les compositions peuvent se présenter sous forme de dispersion vésiculaire de lipides amphiphiles ioniques ou non-ioniques. Elles sont alors préparées notamment en faisant gonfler les lipides dans une solution aqueuse pour former des sphérules dispersées dans le milieu aqueux comme décrit dans STANDISH & WATKINS, J. Mol. Biol., 13,238 (1965) ou dans les brevets FR-A-2.315.991 et FR-A-2.416.008 de la demanderesse. Les différents types de procédés de préparation sont décrits dans "Les liposomes en biologie cellulaire et pharmacologie", Edition INSERM/John Libery Eurotext, 1987, pages 6 à 18.

Le pH des compositions selon l'invention est généralement compris entre 3 et 9 et de préférence entre 3 et 6.

Les flavonoïdes peuvent être ajoutés à la composition juste avant l'emploi; ils sont donc , dans ce cas, conditionnés à part des autres ingrédients de la composition.

On va maintenant donner à titre d'illustration plusieurs exemples selon l'invention.

**EXEMPLE 1**

On a préparé une lotion de soin non rincée de composition suivante :

| | |
|---|---|
| - épicatéchine | 1, 04 g |
| - acétone | 20, 83 g |
| - eau qsp | 100 g |

Cette lotion a été appliquée sur des cheveux fortement décolorés, et on a ensuite mesuré la vitesse de gonflement dans l'eau du cheveu ainsi traité.

La vitesse de gonflement représente la vitesse de pénétration de l'eau dans les cheveux. La variation de cette vitesse, mesurée avant et après un traitement du cheveu, permet de caractériser les effets du traitement appliqué : une augmentation de la vitesse de gonflement caractérise une dégradation des cheveux, alors qu'une diminution de la vitesse de gonflement confirme la pénétration dans les cheveux de molécules de taille suffisamment petites apportées par le traitement appliqué.

Sur un cheveu traité avec la lotion selon l'invention, on a mesuré à l'aide d'un capteur relié à un enregistreur, l'épaisseur initiale $E_i$ du cheveu. On a déposé ensuite 0, 1 ml d'eau sur le cheveu à l'aide d'une seringue ; le gonflement du cheveu se produit. Le capteur mesure l'épaisseur du cheveu pendant toute la phase de gonflement. A la fin du phénomène, on a mesuré l'épaisseur finale $E_f$.

La figure 1 montre la courbe d'enregistrement obtenue après une opération de mesure : elle donne la variation de l'épaisseur (E) du cheveu en fonction du temps (t).

Pour déterminer le temps de gonflement $t_G$, on a tracé la pente à l'origine (p) de la courbe ; l'intersection de cette pente avec la droite au niveau de l'épaisseur finale détermine l'instant de la fin du phénomène de gonflement.

La vitesse de gonflement est alors déterminée par la relation :

$$\text{vitesse de gonflement} = (E_f - E_i)/t_G$$

On a comparé la vitesse de gonflement d'un cheveu traité avec la lotion selon l'invention à celle d'un cheveu non traité. On a constaté que la vitesse de gonflement du cheveu traité selon l'invention avait diminué de 49, 9 % par rapport à celle du cheveu non traité.

**EXEMPLE 2**

On a préparé une lotion de soin non rincée de composition suivante :

| | |
|---|---|
| - taxifoline | 1,04 g |
| - acétone | 20,83 g |
| - eau qsp | 100 g |

Cette lotion a été appliquée sur des cheveux fortement décolorés et on a mesuré la vitesse de gonflement dans l'eau comme décrit à l'exemple 1. On a constaté que la vitesse de gonflement du cheveu traité selon l'invention avait diminué de 73, 4 % par rapport à celle du cheveu non traité.

**Revendications**

1. Utilisation, dans une composition cosmétique, de flavonoïdes pour préserver et/ou renforcer au moins une propriété mécanique des cheveux choisie parmi la résistance à la traction, l'élasticité, la résistance au gonflement dans un milieu aqueux ou la charge à la rupture, face à l'action répétée de traitements capillaires, les flavonoïdes étant choisis parmi les flavanones, les flavones, les flavonols, les dihydroflavonols, les catéchines et les leucoanthocyanidines.

2. Utilisation selon la revendication précédente, caractérisée par le fait que les flavonoïdes sont choisis parmi les composés de formule (I) :

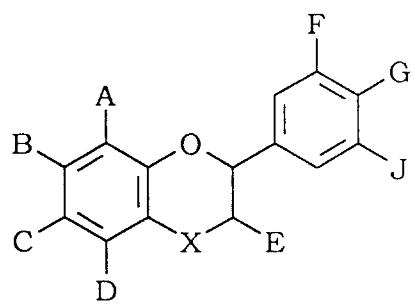

(I)

ou (II) :

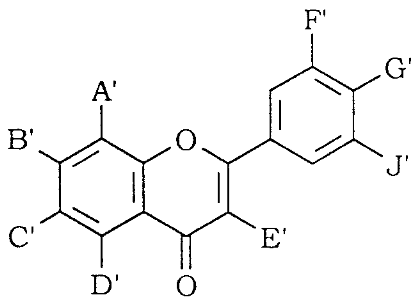

(II)

dans lesquelles :

A, B, C et D, indépendamment l'un de l'autre, représentent H ou OH,
E représente H, OH, ou OR, où R représente :

R = 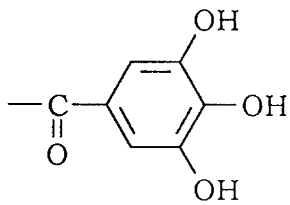

F, G, J représentent, indépendamment l'un de l'autre, H ou OH ; et X représente :

X = 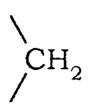 , 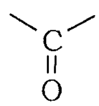 , 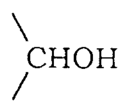

A', C' et D', indépendamment l'un de l'autre, représentent H, OH ou $OCH_3$,
E' repésente H , OH ou OR', où R' représente un radical d'un sucre,
B', F', G' et J', indépendamment l'un de l'autre, représentent H, OH, $OCH_3$ ou $OCH_2$-$CH_2$-OH.

3. Utilisation selon la revendication 2, caractérisée par le fait que les composés de formule (I) sont choisis parmi la taxifoline, la catéchine, l'épicatéchine, l'ériodictyol, la naringénine, l'épigallocatéchine et le gallate de l'épigallocatéchine.

4. Utilisation selon la revendication 2, caractérisée par le fait que les composés de formule (II) sont choisi parmi la rutine, la troxérutine, la chrysine, la tangérétine et la lutéoline.

5. Utilisation selon l'une quelconque des revendications précédentes, caractérisée par le fait que les flavonoïdes sont présents à une concentration comprise entre 0, 001 % et 10 % et de préférence entre 0, 005 et 5 % en poids dans un support cosmétiquement acceptable.

6. Utilisation selon l'une quelconque des revendications précédentes, caractérisée en ce que la composition cosmétique contenant les flavonoïdes se présente sous forme de lotion aqueuse ou hydroalccolique, de gel, de crème, d'émulsion, de dispersion vésiculaire, de mousse, de spray.

7. Utilisation selon l'une quelconque des revendications précédentes, caractérisée en ce que la composition cosmétique contenant les flavonoïdes se présente sous forme de shampooing, d'après-shampooing à rincer ou non, de composition pour permanente, défrisage, coloration ou décoloration, ou encore sous forme de composition à rincer, à appliquer avant ou après une coloration, une permanente ou un défrisage ou encore entre les deux étapes d'une permanente ou d'un défrisage.

8. Utilisation selon l'une quelconque des revendications précédentes, caractérisée en ce que la composition cosmétique contenant les flavonoïdes contient au moins un additif cosmétique.

9. Utilisation selon l'une quelconque des revendications précédentes, caractérisée en ce que la composition cosmétique ne contient pas d'extrait de ginkgo.

## Claims

1. Use, in a cosmetic composition, of flavonoids for preserving and/or enhancing at least one mechanical property of the hair, chosen from tensile strength, elasticity, resistance to swelling in an aqueous medium or the breaking load, in relation to the repeated action of hair treatment, the flavonoids being chosen from flavanones, flavones, flavonols, dihydroflavonols, catechins and leucoanthocyanidins.

2. Use according to the preceding claim, characterized by the fact that the flavonoids are chosen from the compounds of formula (I):

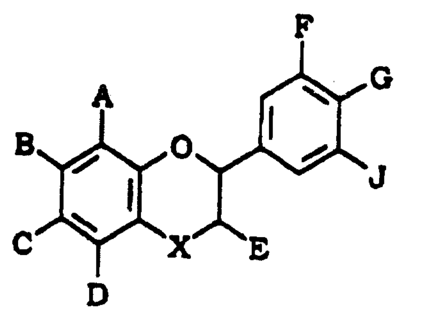

(I)

or (II):

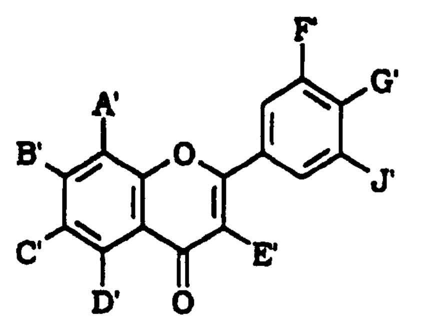

(II)

in which:

A, B, C and D, independently of each other represent H or OH,
E represents H, OH, or OR, where R represents:

R =
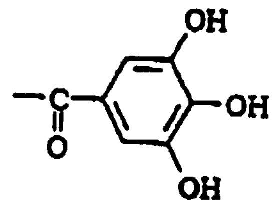

F, G, J represent, independently of each other, H or OH; and X represents:

X =
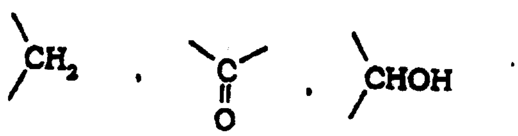

A', C' and D', independently of each other, represent H, OH or $OCH_3$,
E' represents H, OH or OR', where R' represents a radical of a sugar,
B', F', G' and J', independently of each other, represent H, OH, $OCH_3$ or $OCH_2$-$CH_2$-OH.

3. Use according to Claim 2, characterized by the fact that the compounds of formula (I) are chosen from taxifolin, catechin, epicatechin, eriodictyol, naringenin, epigallocatechin and epigallocatechin gallate.

4. Use according to Claim 2, characterized by the fact that the compounds of formula (II) are chosen from rutin, troxerutin, chrysin, tangeretin and luteolin.

5. Use according to any one of the preceding claims, characterized by the fact that the flavonoids are present at a concentration of between 0.001% and 10%, and preferably of between 0.005 and 5% by weight, in a cosmetically acceptable carrier.

6. Use according to any one of the preceding claims, characterized in that the cosmetic composition containing the flavonoids is provided in the form of an aqueous or aqueous-alcoholic lotion, a gel, a cream , an emulsion, a vesicular dispersion, a foam, a spray.

7. Use according to any one of the preceding claims, characterized in that the cosmetic composition containing the flavonoids is provided in the form of a shampoo, a rinse-off or leave-in conditioner, a composition for permanent waving, hair straightening, dyeing or bleaching, or alternatively in the form of a rinse-off composition to be applied before or after a dyeing, a permanent waving or a hair straightening or alternatively between the two stages of a permanent waving or a hair straightening.

8. Use according to any one of the preceding claims, characterized in that the cosmetic composition containing the flavonoids contains at least one cosmetic additive.

9. Use according to any one of the preceding claims, characterized in that the cosmetic composition does not contain any ginkgo extract.

**Patentansprüche**

1. Verwendung von Flavonoiden in einer kosmetischen Zusammensetzung, um mindestens eine mechanische Eigenschaft des Haars, die unter der Zugfestigkeit, der Elastizität, der Quellfestigkeit in einem wässerigen Medium oder der Reißfestigkeit ausgewählt ist, bei wiederholten Haarbehandlungen zu bewahren und/oder zu verbessern,

wobei die Flavonoide unter den Flavanonen, Flavonen, Flavonolen, Dihydroflavonolen, Catechinen und Leukoanthocyanidinen ausgewählt sind.

2. Verwendung nach dem vorhergehenden Anspruch, dadurch gekennzeichnet, daß die Flavonoide ausgewählt sind unter den Verbindungen der Formel (I):

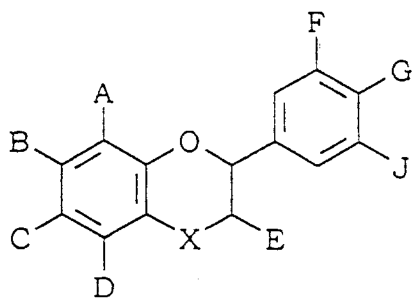

(I)

oder (II):

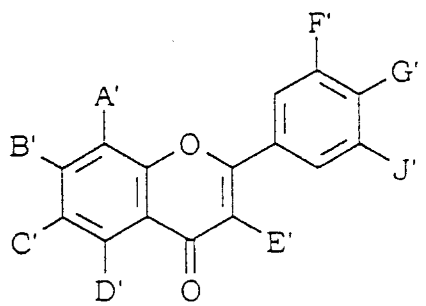

(II),

worin bedeuten:

- A, B, C und D, unabhängig voneinander, H oder OH,
- E H, OH oder OR, worin R:

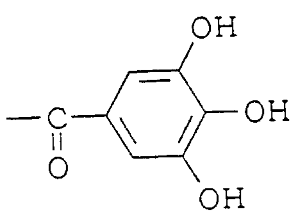

bedeutet,
- F, G und J, unabhängig voneinander, H oder OH und
- X

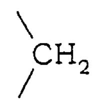 , 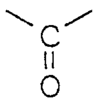 , 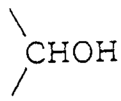 ,

- A', C' und D', unabhängig voneinander, H, OH oder $OCH_3$,
- E' H, OH oder OR', worin R' eine Zuckergruppe bedeutet,
  und
- B', F', G' und J', unabhängig voneinander, H, OH, $OCH_3$ der $OCH_2$-$CH_2$-OH.

**3.** Verwendung nach Anspruch 2, dadurch gekennzeichnet, daß die Verbindungen der Formel (I) unter Taxifolin, Catechin, Epicatechin, Eriodictyol, Naringenin, Epigallocatechin und Epigallocatechingallat ausgewählt sind.

**4.** Verwendung nach Anspruch 2, dadurch gekennzeichnet, daß die Verbindungen der Formel (II) unter Rutin, Troxerutin, Chrysin, Tangeretin und Luteolin ausgewählt sind.

**5.** Verwendung nach einem der vorhergehenden Ansprüche, dadurch gekennzeichnet, daß die Flavonoide in einer Konzentration im Bereich von 0,001 bis 10 Gew.-% und vorzugsweise von 0,005 bis 5 Gew.-% in einem kosmetisch akzeptablen Träger vorliegen.

**6.** Verwendung nach einem der vorhergehenden Ansprüche, dadurch gekennzeichnet, daß die kosmetische Zusammensetzung, die die Flavonoide enthält, in Form einer wässerigen oder wässerig-alkoholischen Lotion, eines Gels, einer Creme, einer Emulsion, einer Vesikeldispersion, eines Schaums oder eines Sprays vorliegt.

**7.** Verwendung nach einem der vorhergehenden Ansprüche, dadurch gekennzeichnet, daß die kosmetische Zusammensetzung, die die Flavonoide enthält, als Haarshampoo, als Zusammensetzung zur Anwendung nach der Haarwäsche, die im Haar verbleibt oder ausgespült wird, als Zusammensetzung zum Dauerwellen, Entkräuseln, Färben oder Entfärben, oder auch als Zusammensetzung zum Ausspülen, zur Anwendung vor oder nach einer Färbung, einer Dauerwelle oder einer Entkräuselung oder auch zur Anwendung zwischen den beiden Schritten einer Dauerwelle oder einer Entkräuselung vorliegt.

**8.** Verwendung nach einem der vorhergehenden Ansprüche, dadurch gekennzeichnet, daß die kosmetische Zusammensetzung, die die Flavonoide enthält, mindestens einen kosmetischen Zusatzstoff enthält.

**9.** Verwendung nach einem der vorhergehenden Ansprüche, dadurch gekennzeichnet, daß die kosmetische Zusammensetzung keinen Gingkoextrakt enthält.

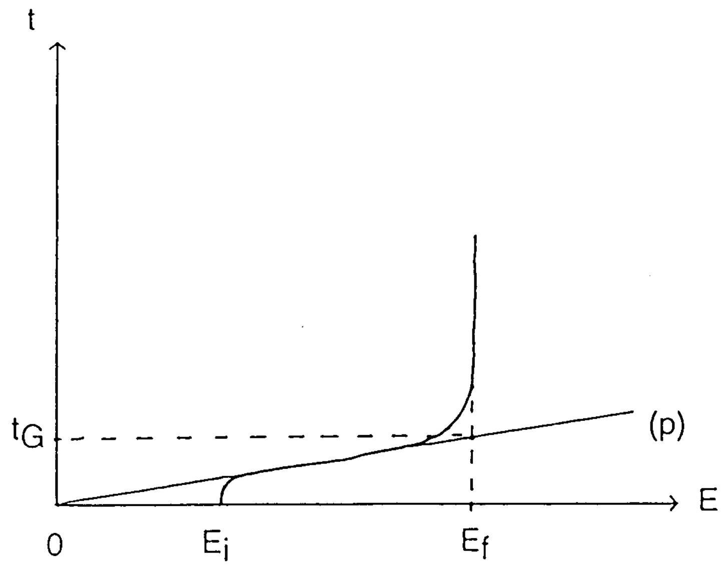


Figure 1